# EUROPEAN PATENT SPECIFICATION

(11) **EP 3 334 411 B1**
(45) Date of publication and mention of the grant of the patent: **29.09.2021**
(21) Application number: 16767361.5
(22) Date of filing: 10.08.2016
(51) Int. Cl.: A61K 31/728, A61K 31/10, A61K 9/00, A61P 19/02, A61K 47/02

(54) **INJECTABLE LIQUID COMPOSITION FOR USE IN THE INTRA-ARTICULAR INFILTRATION THERAPY**
INJIZIERBARE FLÜSSIGE ZUSAMMENSETZUNG ZUR VERWENDUNG IN DER INTRAARTIKULÄREN INFILTRATIONSTHERAPIE
COMPOSITION LIQUIDE INJECTABLE POUR UTILISATION DANS LA THÉRAPIE PAR INFILTRATION INTRA-ARTICULAIRE

(30) Priority: 13.08.2015 IT UB20153110
(43) Date of publication of application: 20.06.2018
(73) Proprietor: Alfakjn S.r.l., 27026 Garlasco (PV) (IT)
(72) Inventor: FERRARI, Alessio, 27026 Garlasco (PV) (IT)
(74) Representative: Hoffmann Eitle
(86) International application number: PCT/IB2016/054809
(87) International publication number: WO 2017/025903

(56) References cited:
- WO-A1-2010/049898
- WO-A1-2014/032804
- WO-A1-2015/092516
- US-A1- 2008 102 107
- GIGANTE A ET AL: "The role of intra-articular hyaluronan (Sinovial(R)) in the treatment of osteoarthritis", RHEUMATOLOGY INTERNATIONAL: CLINICAL AND EXPERIMENTAL INVESTIGATIONS, SPRINGER, DE, vol. 31, no. 4, 1 January 2011 (2011-01-01), pages 427-444, XP009162777, ISSN: 0172-8172
- USHA P R ET AL: "Randomised, double-blind, parallel, placebo-controlled study of oral glucosamine, methylsulfonylmethane and their combination in osteoarthritis", CLINICAL DRUG INVESTIGATION, vol. 24, no. 6, 2004, pages 353-363, XP008182403, ISSN: 1173-2563

## Description

The present invention relates to an injectable liquid composition which comprises an effective amount of a mixture comprising or, alternatively, consisting of hyaluronic acid (HA), or a pharmaceutically acceptable salt thereof, methylsulfonylmethane (MSM), and a physiologically acceptable carrier; said composition being for use in the intra-articular infiltration therapy, wherein said hyaluronic acid, or a pharmaceutically acceptable salt thereof, has an average molecular weight comprised from 800 KDa to 1200 KDa, and the methylsulfonylmethane is in an amount by weight comprised from 0.5% to 8%, relative to the total weight of the composition; wherein said composition is for use in the intra-articular and periarticular treatment of rheumatic diseases. In particular, the present invention relates to said injectable liquid composition for use in the treatment of osteoarthritis of the knee.

The intra-articular use of hyaluronic acid is known. Hyaluronic acid is a glycosaminoglycan being able to bind several molecules of water and is usually found in biological tissues with trophic and hydrating function. At intra-articular level, the hyaluronic acid or a salt thereof play several functions, among which: viscoelastic lubrication, support to the trophism of cartilage and intra-articular biochemical reestablishment. The injection of hyaluronic acid directly in the articular space allows to restoring the synovial fluid, favoring the persistence *in situ* of the substance and the subsequent therapeutic response.

The term "viscosupplementation" means, indeed, restoring the viscoelastic properties of synovial fluids such as joint damping, lubrication and elasticity, whereas the term biosupplementation relates to the restoring of the articular rheology with anti-inflammatory and anti-nociceptive effects, normalization of the endogenous hyaluronic acid synthesis and chondroprotection.

WO2014/032804 discloses injectable liquid compositions comprising 0,2-5% w/w hyaluronic acid having molecular weight 100-3500 kDa in combination with 0,001-5% w/w MSM which may be used for viscosupplementation and in particular for the intra-articular treatment of rheumatic diseases.

However, there is a need from the operators in the field for having a composition based on hyaluronic acid (and/or a pharmaceutically acceptable salt thereof) which is more effective, active also against limb swelling and without causing severe burning sensation, upon injection, in order to extend the use thereof to a larger kind of patients.

Nevertheless, what is unavailable to operators thus far, but that generates a great interest, is to be able to have a composition based on hyaluronic acid (and/or a pharmaceutically acceptable salt thereof) being effective in readily reducing the pain and inflammation of the limb, as well as a composition which can also act in the reduction of painful muscular spasms.

The Applicant, following to a long and intense research and development activity, solved the above-cited limits and drawbacks of the known technique by developing a novel injectable liquid composition based on hyaluronic acid (and/or a pharmaceutically acceptable salt thereof) and comprising a physiologically acceptable carrier.

It is an object of the present invention an injectable liquid composition comprising an effective amount of a mixture which comprises or, alternatively, consists of hyaluronic acid (and/or a pharmaceutically acceptable salt thereof) and methylsulfonylmethane, and a physiologically acceptable carrier; said composition being for use in the intra-articular infiltration therapy, having the characteristics as set forth in the appended claims.

Preferred embodiments of the present invention will be hereinafter described in detail.

The Applicant found to be useful and very advantageous the development of an injectable liquid composition, which comprises an effective amount of a mixture comprising or, alternatively, consisting of hyaluronic acid and/or a pharmaceutically acceptable salt thereof and methylsulfonylmethane, and a physiologically acceptable carrier, as disclosed in the appended claims. Preferably, said carrier can be either in liquid or solid form. In an embodiment, the carrier is in liquid form (liquid carrier) and is physiologically acceptable since it contains, for example, pharmaceutically acceptable liquid vehicles (water for injections), formulation additives, diluents and excipients. Preferably, said carrier in liquid form comprises a pyrogen-free sodium chloride and/or disodium hydrogen phosphate anhydrous -Na₂HPO₄, and/or sodium dihydrogen phosphate dihydrate -NaH₂PO₄x2H₂O, and/or water for injections.

Said injectable liquid composition of the present invention showed several technical advantages relative to an injectable liquid composition based on hyaluronic acid alone, or a pharmaceutically acceptable salt thereof.

The injectable liquid composition of the present invention is for use in the intra-articular infiltration therapy in the intra-and periarticular treatment of rheumatic diseases; in particular said composition is for use in the treatment of the hip, knee, shoulder, articular and cervical facets and small joints of the hand and foot. Advantageously, said composition is for use in the treatment of osteoarthritis of the knee, by intra-articular injection.

The hyaluronic acid, or a pharmaceutically acceptable salt thereof, is an unbranched, straight-chain hyaluronic acid, preferably of biofermentative origin.

The hyaluronic acid, or a pharmaceutically acceptable salt thereof, has an average molecular weight comprised from 800 KDa to 1200 KDa, preferably 1000 KDa. It was observed that this range of molecular weight ensures a suitable affinity for hyaluronic acid receptors and a better stimulation of the hyaluronic acid biosynthesis.

A pharmaceutically acceptable salt of hyaluronic acid is sodium hyaluronate.

The composition of the present invention contains hyaluronic acid, and/or a pharmaceutically acceptable salt thereof, in an amount by weight comprised from 0.5% to 3%, preferably from 1% to 2%, relative to the total weight of the composition. The composition of the present invention contains methylsulfonylmethane (MSM) in an amount by weight comprised from 0.5% to 8%, preferably from 1% to 5%, relative to the total weight of the composition.

It was noted that a composition comprising an amount of MSM within these ranges allows to optimizing the efficacy of the infiltration therapy and minimizing the side effects, in particular the burning sensation upon injection.

Furthermore, it was observed that the injectable liquid composition of the present invention comprising MSM in an amount by weight comprised from 0.5% to 8%, preferably from 1% to 5%, relative to the total weight of the composition and hyaluronic acid, or a pharmaceutically acceptable salt thereof, having an average molecular weight comprised from 800 KDa to 1200 KDa, preferably 1000 KDa, when stored for at least 6 months under accelerated stability conditions (temperature = 40°C, 75% relative humidity) exhibited no significant changes neither in pH nor in the amount of bacterial endotoxins.

It was also found that MSM at the above-cited amount has an antioxidant effect and increases the hyaluronic acid stability against free radical stress, thermal stress and UV stress.

The injectable liquid composition of the present invention maintains stable and clear over time and gives rise to no deposit when stored in a syringe for injection over a 24-month period.

The liquid composition of the present invention is preferably buffered to a pH value comprised from 6.5 to 7.5.

In a preferred embodiment, the injectable liquid composition of the present invention comprises, for example, for a total volume of the composition equal to 2 ml:
- Sodium hyaluronate HTL-SV6 (Code MP 1967): 32.00 mg
- Methylsulfonylmethane (MSM): 100 mg
- Pyrogen-free sodium chloride (Code MP0052): 17.00 mg
- Disodium hydrogen phosphate anhydrous -Na₂HPO₄ (Code MP2017): 0.32 mg
- Sodium dihydrogen phosphate dihydrate -NaH₂PO₄ x 2H₂O (Code MP1887): 0.090 mg
- Water for injections: q.s. to 2 ml

### Experimental part

A clinical trial protocol was developed in order to assess a novel class III medical device (the injectable liquid composition object of the present invention), containing Proakjn™ based on hyaluronic acid and methylsulfonylmethane -MSM (the mixture object of the present invention) in the treatment of osteoarthritis of the knee. 20 subjects, both males and females, between 18 and 75 years old, diagnosed with a radiologically-confirmed osteoarthritis of the knee were enrolled. The 20 patients were assessed with reference to the 6 criteria listed below. At least four of the following six criteria had to be met:
(1) erythrocyte sedimentation rate < 30 millimeter/h;
(2) rheumatoid factor level <1:160;
(3) morning stiffness for a period no longer than 30 minutes;
(4) crepitus on active movement;
(5) softness of bone margins; and
(6) medical assessment of the absence of rheumatic disease.

The trial protocol contemplates the treatment of 10 of the above 20 subjects (Reference Group 1) with intra-articular injections of straight-chain hyaluronic acid with a molecular weight comprised between 800 and 1200 KDa at a concentration of 1.6% equal to 32 mg HA in 2 ml of sterile saline. The remaining 10 of the above 20 subjects (Group 2) were treated with intra-articular injections of Proakjn™ based on straight-chain hyaluronic acid with a molecular weight comprised between 800 and 1200 KDa at a concentration of 1.6% equal to 32 mg HA in 2 ml of sterile saline supplemented with MSM 100 mg - liquid carrier (the injectable liquid composition object of the present invention).

The composition tested in the present trial in patients of Reference Group 1, in the form of a pre-filled syringe of 2 ml volume, has the following quali-quantitative characteristics (Reference Composition):
- Sodium hyaluronate HTL-SV6 (Code MP 1967): 32.00 mg
- Pyrogen-free sodium chloride (Code MP0052): 17.00 mg
- Disodium hydrogen phosphate anhydrous -Na₂HPO₄ (Code MP2017): 0.32 mg
- Sodium dihydrogen phosphate dihydrate -NaH₂PO₄x2H₂O (Code MP1887): 0.090 mg
- Water for injections: q.s. to 2 ml

The composition tested in the present trial in patients of Group 2, in the form of a pre-filled syringe of 2 ml volume, has the following quali-quantitative characteristics (the injectable liquid composition of the present invention):
- Sodium hyaluronate HTL-SV6 (Code MP 1967): 32.00 mg
- Methylsulfonylmethane (MSM): 100 mg
- Pyrogen-free sodium chloride (Code MP0052): 17.00 mg
- Disodium hydrogen phosphate anhydrous -Na₂HPO₄ (Code MP2017): 0.32 mg
- Sodium dihydrogen phosphate dihydrate -NaH₂PO₄x2H₂O (Code MP1887): 0.090 mg
- Water for injections: q.s. to 2 ml

The double-blind trial (Reference Group 1 and Group 2) was carried out according to the following therapeutic regimen (for both the groups): N.° 1 injection per week for three weeks followed by a follow up 6 months after the last injection.

Before starting the intra-articular injections, the following parameters were measured from the articular fluid: ESR, PCR and Fibrinogen. The VAS value of the pain scale was also assessed by a specialist visit. Patients filled in a self-assessment questionnaire (joint pain at rest, pain at night, pain on movement, level of joint flexibility), for each parameter the subjects assigned a value from 1 to 5, being 1 the lowest value and 5 the highest value.

After the follow up, all the subjects were re-examined and the articular fluid was taken in order to evaluate again the parameters ESR, PCR and Fibrinogen. Furthermore, by a specialist visit, the VAS value of the pain scale was assessed. Finally, the self-assessment questionnaire was distributed again.

It was found that the injectable liquid composition of the present invention, relative to the reference liquid composition, advantageously showed to be more effective and active even against the swelling, to notably reduce both pain and inflammation of the treated limb; furthermore, it resulted very useful in reducing the painful muscle spasms. Most of the subjects from Group 2 reported no burning sensation after injection of the injectable liquid composition of the present invention.

Some of the measured parameters demonstrate that methylsulfonylmethane (MSM) plays an additional and complementary anti-inflammatory and analgesic effect being able to provide to the treated subject a fast relief after injection of the injectable liquid composition of the present invention.

On the medium term (some days) MSM reduces the intra-articular inflammation, thus ameliorating the pain symptoms combined with hyaluronic acid and/or sodium hyaluronate salt, restoring the lubricant ability of synovial fluid.

## Claims

1. An injectable liquid composition which comprises an effective amount of a mixture comprising or, alternatively, consisting of hyaluronic acid, or a pharmaceutically acceptable salt thereof, methylsulfonylmethane, and a physiologically acceptable carrier; said composition being for use in the intra-articular infiltration therapy, wherein said hyaluronic acid, or a pharmaceutically acceptable salt thereof, has an average molecular weight comprised from 800 KDa to 1200 KDa, and the methylsulfonylmethane is in an amount by weight comprised from 0.5% to 8%, relative to the total weight of the composition; wherein said composition is for use in the intra-articular and periarticular treatment of rheumatic diseases.

2. The injectable liquid composition for use according to claim 1, wherein said composition is for use in the treatment of the hip, knee, shoulder, articular and cervical facets and small joints of the hand and foot.

3. The injectable liquid composition for use according to claim 2, wherein said composition is for use in the treatment of osteoarthritis of the knee, by intra-articular injection.

4. The injectable liquid composition for use according to any one of the preceding claims, wherein said hyaluronic acid, or a pharmaceutically acceptable salt thereof, is unbranched, straight-chain hyaluronic acid, of biofermentative origin.

5. The injectable liquid composition for use according to any one of the preceding claims, wherein said hyaluronic acid, or a pharmaceutically acceptable salt thereof, has an average molecular weight of 1000 KDa.

6. The injectable liquid composition for use according to any one of the preceding claims, wherein said pharmaceutically acceptable salt is sodium hyaluronate.

7. The injectable liquid composition for use according to any one of the preceding claims, wherein the hyaluronic acid or a pharmaceutically acceptable salt thereof is in an amount by weight comprised from 0.5% to 3%, preferably from 1% to 2%, relative to the total weight of the composition.

8. The injectable liquid composition for use according to any one of the preceding claims, wherein the methylsulfonylmethane is in an amount by weight comprised from 1% to 5%, relative to the total weight of the composition.

9. The injectable liquid composition for use according to any one of the preceding claims, wherein the composition is buffered to a pH value comprised from 6.5 to 7.5, preferably said injectable liquid composition comprises, for example, for a total volume of the composition equal to 2 ml:
- Sodium hyaluronate HTL-SV6 (Code MP 1967): 32.00 mg
- MSM: 100 mg
- Pyrogen-free sodium chloride (Code MP0052): 17.00 mg
- Disodium hydrogen phosphate anhydrous -Na₂HPO₄ (Code MP2017): 0.32 mg
- Sodium dihydrogen phosphate dihydrate -NaH₂PO₄x2H₂O (Code MP1887): 0.090 mg
- Water for injections: q.s. to 2 ml.

## Patentansprüche

1. Injizierbare flüssige Zusammensetzung, die eine wirksame Menge eines Gemisches umfasst, umfassend oder alternativ bestehend aus Hyaluronsäure oder einem pharmazeutisch annehmbaren Salz davon, Methylsulfonylmethan und einem physiologisch annehmbaren Träger; worin die Zusammensetzung zur Verwendung bei der intraartikulären Infiltrationstherapie bestimmt ist, worin die Hyaluronsäure oder ein pharmazeutisch annehmbares Salz davon ein durchschnittliches Molekulargewicht von 800 KDa bis 1200 KDa aufweist und das Methylsulfonylmethan in einer Gewichtsmenge von 0,5% bis 8%, bezogen auf das Gesamtgewicht der Zusammensetzung, vorliegt; worin die Zusammensetzung zur Verwendung bei der intraartikulären und periartikulären Behandlung von rheumatischen Erkrankungen bestimmt ist.

2. Injizierbare flüssige Zusammensetzung zur Verwendung gemäß Anspruch 1, worin die Zusammensetzung zur Verwendung bei der Behandlung der Hüfte, des Knies, der Schulter, der Facettengelenke und kleinen Gelenke der Hand und des Fußes bestimmt ist.

3. Injizierbare flüssige Zusammensetzung zur Verwendung gemäß Anspruch 2, worin die Zusammensetzung zur Verwendung bei der Behandlung der Osteoarthritis des Knies durch intraartikuläre Injektion bestimmt ist.

4. Injizierbare flüssige Zusammensetzung zur Verwendung gemäß einem der vorhergehenden Ansprüche, worin die Hyaluronsäure oder ein pharmazeutisch annehmbares Salz davon unverzweigte, geradkettige Hyaluronsäure biofermentativen Ursprungs ist.

5. Injizierbare flüssige Zusammensetzung zur Verwendung gemäß einem der vorhergehenden Ansprüche, worin die Hyaluronsäure oder ein pharmazeutisch annehmbares Salz davon ein durchschnittliches Molekulargewicht von 1000 KDa aufweist.

6. Injizierbare flüssige Zusammensetzung zur Verwendung gemäß einem der vorhergehenden Ansprüche, worin das pharmazeutisch annehmbare Salz Natriumhyaluronat ist.

7. Injizierbare flüssige Zusammensetzung zur Verwendung gemäß einem der vorhergehenden Ansprüche, worin die Hyaluronsäure oder ein pharmazeutisch annehmbares Salz davon in einer Gewichtsmenge von 0,5% bis 3%, vorzugsweise von 1% bis 2%, bezogen auf das Gesamtgewicht der Zusammensetzung, vorliegt.

8. Injizierbare flüssige Zusammensetzung zur Verwendung gemäß einem der vorhergehenden Ansprüche, worin das Methylsulfonylmethan in einer Gewichtsmenge von 1 bis 5%, bezogen auf das Gesamtgewicht der Zusammensetzung, vorliegt.

9. Injizierbare flüssige Zusammensetzung zur Verwendung gemäß einem der vorhergehenden Ansprüche, worin die Zusammensetzung auf einen pH-Wert von 6,5 bis 7,5 gepuffert ist, vorzugsweise umfasst die injizierbare flüssige Zusammensetzung für ein Gesamtvolumen der Zusammensetzung von 2 ml beispielsweise:
- Natriumhyaluronat HTL-SV6 (Code MP 1967): 32,00 mg
- MSM: 100 mg
- Pyrogenfreies Natriumchlorid (Code MP0052): 17,00 mg
- Wasserfreies Dinatriumhydrogenphosphat - Na₂HPO₄ (Code MP2017): 0,32 mg
- Natriumdihydrogenphosphat Dihydrat - NaH₂PO₄x2H₂O (Code MP1887): 0,090 mg
- Wasser für Injektionszwecke: q.s. auf 2 ml.

## Revendications

1. Composition liquide injectable qui comprend une quantité efficace d'un mélange comprenant ou, en variante, consistant en, de l'acide hyaluronique, ou un sel pharmaceutiquement acceptable de celui-ci, du méthylsulfonylméthane, et un véhicule physiologiquement acceptable ; ladite composition étant destinée à être utilisée dans une thérapie d'infiltration intra-articulaire, dans laquelle ledit acide hyaluronique, ou un sel pharmaceutiquement acceptable de celui-ci, a un poids moléculaire moyen compris de 800 kDa à 1200 kDa, et le méthylsulfonylméthane est présent en une quantité en poids comprise de 0,5 % à 8 % par rapport au poids total de la composition ; laquelle composition est destinée à être utilisée dans le traitement intra-articulaire et périarticulaire de maladies rhumatismales.

2. Composition liquide injectable pour une utilisation selon la revendication 1, laquelle composition est destinée à être utilisée dans le traitement de la hanche, du genou, de l'épaule, des facettes articulaires et cervicales, et des petites articulations de la main et du pied.

3. Composition liquide injectable pour une utilisation selon la revendication 2, laquelle composition est destinée à être utilisée dans le traitement de l'arthrose du genou, par injection intra-articulaire.

4. Composition liquide injectable pour une utilisation selon l'une quelconque des revendications précédentes, dans laquelle ledit acide hyaluronique, ou un sel pharmaceutiquement acceptable de celui-ci, est un acide hyaluronique linéaire non ramifié, d'origine biofermentaire.

5. Composition liquide injectable pour une utilisation selon l'une quelconque des revendications précédentes, dans laquelle ledit acide hyaluronique, ou un sel pharmaceutiquement acceptable de celui-ci, a un poids moléculaire moyen de 1 000 kDa.

6. Composition liquide injectable pour une utilisation selon l'une quelconque des revendications précédentes, dans laquelle ledit sel pharmaceutiquement acceptable est le hyaluronate de sodium.

7. Composition liquide injectable pour une utilisation selon l'une quelconque des revendications précédentes, dans laquelle ledit acide hyaluronique, ou un sel pharmaceutiquement acceptable de celui-ci, est présent en une quantité en poids comprise de 0,5 % à 3 %, de préférence de 1 % à 2 %, par rapport au poids total de la composition.

8. Composition liquide injectable pour une utilisation selon l'une quelconque des revendications précédentes, dans laquelle le méthylsulfonylméthane est présent en une quantité en poids comprise de 1 % à 5 % par rapport au poids total de la composition.

9. Composition liquide injectable pour une utilisation selon l'une quelconque des revendications précédentes, laquelle composition est tamponnée à une valeur de pH comprise de 6,5 à 7,5, de préférence laquelle composition liquide injectable comprend, par exemple, pour un volume total de la composition égal à 2 ml :
- hyaluronate de sodium HTL-SV6 (code MP 1967) : 32,00 mg
- MSM : 100 mg
- chlorure de sodium apyrogène (code MP0052) : 17,00 mg
- hydrogénophosphate disodique anhydre -Na₂HPO₄ (code MP2017) : 0,32 mg
- dihydrogénophosphate de sodium dihydraté -NaH₂PO₄x2H₂O (code MP1887) : 0,090 mg
- eau pour injection : q.s. pour 2 ml.
